# EUROPEAN PATENT SPECIFICATION

(11) **EP 3 244 852 B1**
(45) Date of publication and mention of the grant of the patent: **31.10.2018**
(21) Application number: 15820379.4
(22) Date of filing: 10.12.2015
(51) Int. Cl.: A61F 13/00

(54) **CLOSED ABDOMINAL MANIFOLD DRESSING**
GESCHLOSSENER BAUCHVERBAND MIT VERTEILER
PANSEMENT ABDOMINAL FERMÉ À COLLECTEUR

(30) Priority: 14.01.2015 US 201562103439 P
(43) Date of publication of application: 22.11.2017
(60) Divisional application: 18193893.7
(73) Proprietor: KCI Licensing, Inc., San Antonio, TX 78265 (US)
(72) Inventor: SEDDON, James Killingworth, Ferndown, Dorset BH22 9QJ (GB); HALL, Colin John, Poole, Dorset BH15 3LD (GB)
(74) Representative: Cordina, Kevin John
(86) International application number: PCT/US2015/065133
(87) International publication number: WO 2016/114879

(56) References cited:
- WO-A1-2011/112724
- WO-A2-2007/106594
- US-A1- 2010 160 877
- US-A1- 2012 283 674

## Description

### FIELD

This disclosure relates generally to medical treatment systems and, more particularly, but not by way of limitation, to systems, dressings, devices, and methods suitable for treating a tissue site.

### BACKGROUND

Depending on the medical circumstances, reduced pressure may be used for, among other things, reduced-pressure therapy to encourage granulation at a tissue site, draining fluids at a tissue site, closing a wound, reducing edema, promoting perfusion, and fluid management. Further, therapeutic fluids may be instilled or distributed to a tissue site in combination with or in lieu of reduced-pressure therapy. The instillation of such fluids to a tissue site may assist with preventing infection, enhancing healing, and other therapeutic benefits.

Challenges can exist with distributing fluids to and extracting fluids from a tissue site being subjected to reduced-pressure therapy or fluid instillation. For example, tissue sites may vary in volume, size, geometry, orientation, and other factors. Further, access to these tissue sites may be restricted. These and other factors can make extraction of waste fluids from the tissue site and distribution of therapeutic fluids to the tissue site difficult to perform.

Types of tissue sites that may present particular difficulties may include locations such as a peritoneal cavity, and more generally, an abdominal cavity. For example, the abdominal cavity can be prone to complications such as peritonitis, abdominal compartment syndrome, and infections that can inhibit healing. Thus, improvements to treatment systems that may adapt to various types of tissue sites and orientations, reduce the invasiveness of the treatment, and increase efficiency and healing times may be desirable.

US 2012/283674 A1, WO 2007/106594 A2, US 2010/160877 A1 and WO 2011/112724 A1 describe reduced pressure treatment devices.

### SUMMARY

There is provided a dressing for treating a tissue site according to claim 1. A selection of optional features is defined in the dependent claims. Shortcomings with certain aspects of tissue treatment systems, dressings, devices, and methods are addressed as shown and described in a variety of illustrative, non-limiting embodiments herein.

As described herein a system for treating a tissue site may include a manifold, a blade, a reduced-pressure lumen, and a reduced-pressure source. The manifold may include an insertion end that may be adapted for insertion at the tissue site. The blade may be positioned proximate to the insertion end of the manifold. The blade may include at least one surface feature extending from the manifold toward a periphery of the blade. The surface feature may provide a fluid pathway toward the manifold. The reduced-pressure lumen may be configured for coupling in fluid communication with the manifold. The reduced-pressure source may be configured for coupling in fluid communication with the reduced-pressure lumen. The surface feature may extend to the periphery of the blade. Further, the blade may not enclose foam. Further, in some embodiments, the blade may be formed from a single layer of a substantially liquid impermeable film. Further, in some embodiments, the fluid pathway may be configured to direct fluid along a surface of the blade.

As described herein, a system for treating a tissue site may include an elongate manifold, a paddle, a tail, a reduced-pressure lumen, and a reduced-pressure source. The elongate manifold may be adapted to distribute fluid and may include an insertion end, a connection end, and a length between the insertion end and the connection end. The insertion end of the elongate manifold may be adapted for insertion at the tissue site. The paddle may include at least one surface protrusion, and the paddle may be positioned in fluid communication with the elongate manifold proximate to the insertion end. The tail may be proximate to the connection end of the elongate manifold. Further, the tail may be adapted to be positioned proximate an external opening at the tissue site. The reduced-pressure lumen may be in fluid communication with the elongate manifold. The reduced-pressure source may be for coupling in fluid communication with the reduced-pressure lumen.

In some illustrative embodiments, a dressing for treating a tissue site may include an elongate manifold, a paddle, and a tail. The elongate manifold is surrounded by a manifold film and adapted to distribute fluid. The elongate manifold includes an insertion end, a connection end, and a length between the insertion end and the connection end. The insertion end of the elongate manifold is adapted for insertion at the tissue site. The paddle is positioned in fluid communication with the elongate manifold proximate to the insertion end of the elongate manifold. The paddle includes a paddle film layer extending outward from the elongate manifold and normal relative to the length of the elongate manifold. The tail may be positioned proximate to the connection end of the elongate manifold. Further, the tail may be adapted to be positioned proximate to an external opening at the tissue site.

In some illustrative embodiments, a dressing for treating a tissue site may include a manifold and a paddle. The paddle may include a paddle film layer, a paddle surface, and a plurality of surface protrusions. The paddle film layer may extend outward from the manifold. The paddle surface may be defined by the paddle film layer, and the paddle surface may be in fluid communication with the manifold. The plurality of surface protrusions may be carried by the paddle film layer. Further, the plurality of surface protrusions may be in fluid communication with the manifold, and each of the plurality of surface protrusions may include a protrusion height positioned normal to a protrusion length. The protrusion height may extend outward from the paddle surface, and the protrusion length may extend substantially parallel to the paddle surface. In some illustrative embodiments, the manifold may be adapted to distribute fluid and may be covered by a liquid impermeable material.

As described herein, a method for treating a tissue site may include positioning a dressing adjacent to the tissue site. The dressing may include a manifold and a paddle that may extend outward from the manifold and in fluid communication with the manifold. The paddle may carry at least one surface protrusion on a paddle surface of the paddle. Further, the method may include applying reduced pressure to the tissue site through the manifold and the paddle; moving a fluid from the tissue site to the manifold along the at least one surface protrusion and the paddle surface of the paddle; and extracting the fluid from the tissue site through the manifold.

As described herein, a method for treating an abdominal cavity may include providing a manifold comprising an insertion end adapted for insertion in the abdominal cavity. Further, the method may include providing a blade positioned proximate to the insertion end of the manifold. The blade may include at least one surface feature extending from the manifold toward a periphery of the blade. The surface feature may provide a fluid pathway toward the manifold. The method may further include positioning the manifold and the blade in the abdominal cavity, coupling a reduced-pressure lumen in fluid communication with the manifold, and coupling a reduced-pressure source in fluid communication with the reduced-pressure lumen.

Other aspects, features, and advantages of the illustrative embodiments will become apparent with reference to the drawings and detailed description that follow.

### BRIEF DESCRIPTION OF THE DRAWINGS

FIG. 1 is a partial cut-away view of an illustrative embodiment of a system for treating a tissue site according to this specification;
FIG. 2 is a plan view of an illustrative embodiment of a dressing depicted in the system of FIG. 1;
FIG. 3 is a cross-section of the dressing of FIG. 2, taken along line 3-3 in FIG. 2;
FIG. 4A is a cross-section of the dressing depicted in FIG. 1, taken along line 4A-4A in FIG. 1, illustrating the dressing during the application of instillation fluid to the tissue site; and
FIG. 4B is a cross-section of the dressing depicted in FIG. 1, taken along line 4B-4B in FIG. 1, illustrating the dressing during the application of reduced pressure to the tissue site.

### DETAILED DESCRIPTION OF ILLUSTRATIVE EMBODIMENTS

In the following detailed description of non-limiting, illustrative embodiments, reference is made to the accompanying drawings that form a part hereof. To avoid detail not necessary to enable those skilled in the art to practice the embodiments described herein, the description may omit certain information known to those skilled in the art. The following detailed description is non-limiting, and the scope of the illustrative embodiments are defined by the appended claims. As used herein, unless otherwise indicated, "or" does not require mutual exclusivity.

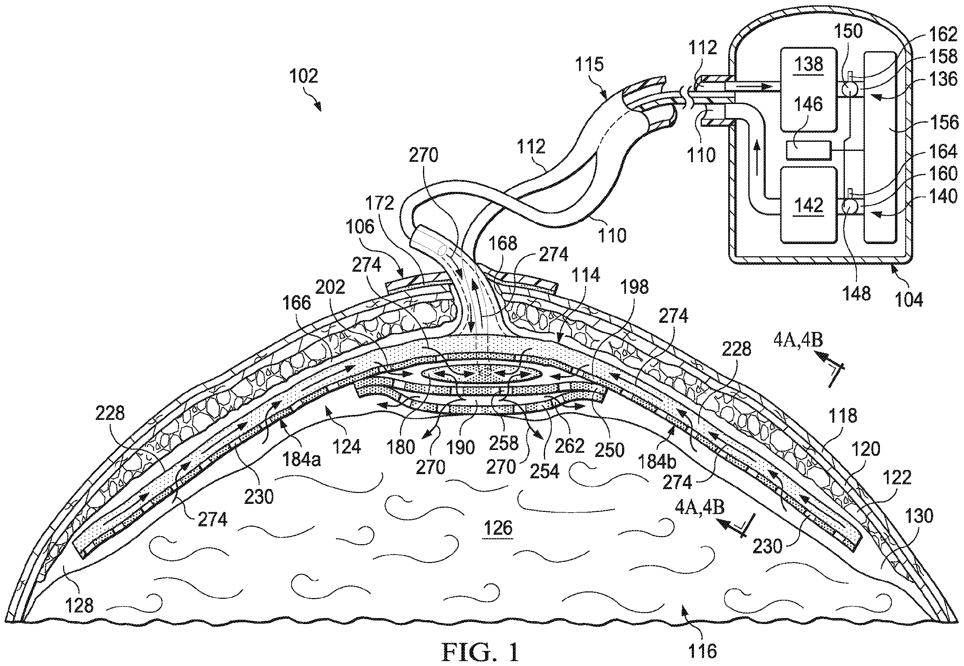

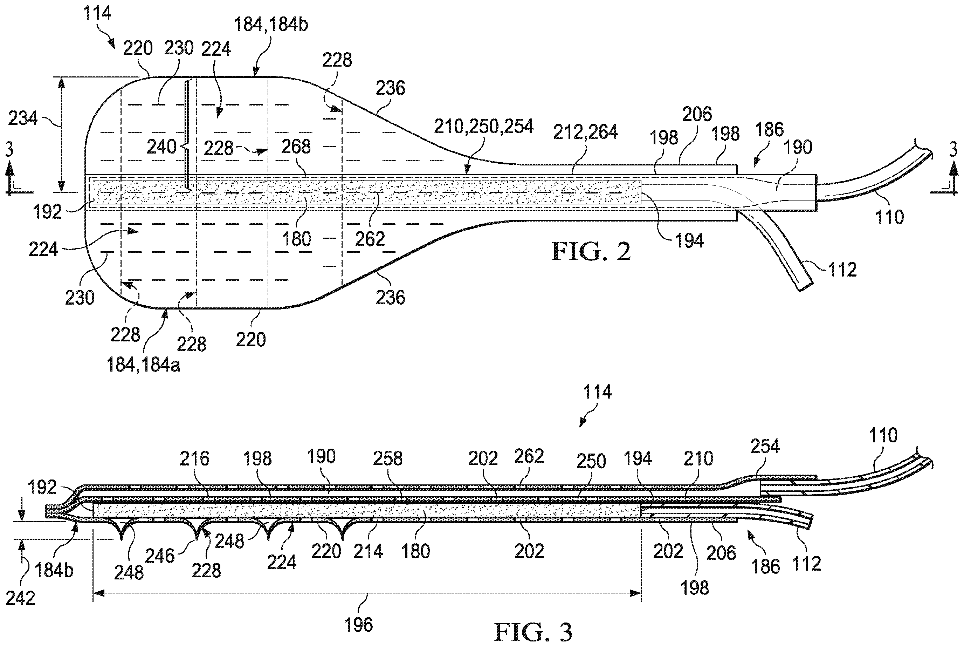

Referring to FIG. 1, in some illustrative embodiments, a system 102 may include a therapy device 104, a dressing sealing member 106, a fluid supply lumen 110, a reduced-pressure lumen 112, and a dressing 114. The fluid supply lumen 110 and the reduced-pressure lumen 112 may be part of a multi-lumen conduit 115. Further, the fluid supply lumen 110 or the reduced-pressure lumen 112 may be supplied as part of the dressing 114 as shown in FIG. 2-3. The system 102 may be suitable for providing fluid instillation or reduced pressure treatment at a tissue site 116. In other embodiments, components of the system 102 may be omitted or added as described herein.

The tissue site 116 may be the bodily tissue of any human, animal, or other organism, including bone tissue, adipose tissue, muscle tissue, dermal tissue, connective tissue, cartilage, tendons, ligaments, or any other tissue. The tissue site 116 may extend through or otherwise involve an epidermis 118, a dermis 120, and a subcutaneous tissue 122. The tissue site 116 may be a sub-surface tissue site as depicted in FIG. 1 that extends below the surface of the epidermis 118. Further, the tissue site 116 may be a surface tissue site (not shown) that predominantly resides on a surface of the epidermis 118.

As shown in FIG. 1, the tissue site 116 may include tissue in a body cavity such as, without limitation, an abdominal cavity 124. The abdominal cavity 124 may include abdominal contents 126 or other tissue proximate the abdominal cavity 124. The dressing 114 may be disposed in the abdominal cavity 124 and supported on a surface of the abdominal contents 126. The dressing 114 may also be positioned in or proximate to a left lateral or first paracolic gutter 128 and a right lateral or second paracolic gutter 130. The first paracolic gutter 128 and the second paracolic gutter 130 may each be, for example, an open space on opposing sides of the abdominal cavity 124 among the abdominal contents 126. The first paracolic gutter 128 may be laterally disposed from the second paracolic gutter 130 or otherwise positioned on an opposite side of the tissue site 116 from the second paracolic gutter 130. Although FIG. 1 depicts the system 102 deployed at the abdominal cavity 124, the system 102 may be used without limitation at other types of tissue sites. Further, the treatment of the tissue site 116 may include, without limitation, the removal of fluids, such as ascites and exudates, reduced-pressure therapy, instillation or distribution of fluids to the tissue site 116, and protection of the tissue site 116.

Continuing with FIG. 1, the therapy device 104 may be for coupling in fluid communication with the fluid supply lumen 110 and the reduced-pressure lumen 112. The fluid supply lumen 110 and the reduced-pressure lumen 112 may be coupled in fluid communication with the dressing 114 as described below. Further, the fluid supply lumen 110 and the reduced-pressure lumen 112 may be combined or formed as part of the multi-lumen conduit 115 shown in FIG. 1. In other embodiments, the fluid supply lumen 110 and the reduced-pressure lumen 112 may be separate conduits, tubes, or pipes, for example.

The therapy device 104 may include a reduced-pressure source 136, a canister 138, a positive-pressure source 140, and a fluid instillation reservoir 142. The canister 138 and the fluid instillation reservoir 142 may each be any suitable containment device for holding a liquid and communicating fluids. Further, in some embodiments, the therapy device 104 may include a controller 146, a positive-pressure valve 148, and a reduced-pressure valve 150 for controlling components of the therapy device 104 as described below. The components of the therapy device 104 may be arranged or associated with one another as shown in FIG. 1 to form the therapy device 104. However, in other embodiments (not shown), the components of the therapy device 104 may be provided separately or independently from the therapy device 104.

The reduced-pressure source 136 may be configured for coupling in fluid communication with the reduced-pressure lumen 112. The canister 138 may be positioned in fluid communication with the reduced-pressure source 136. The reduced-pressure source 136 may be adapted to be coupled in fluid communication with the reduced-pressure lumen 112 through the canister 138. Thus, the canister 138 may have an inlet for receiving reduced pressure from the reduced-pressure source 136 and an outlet for delivering the reduced pressure to the reduced-pressure lumen 112. The reduced-pressure source 136, the reduced-pressure lumen 112, and the canister 136 may be fluidly coupled to one another in any suitable manner, such as, without limitation, through tubing or piping that may be coupled with adhesives, bonding, welding, couplers, or interference fit.

The positive-pressure source 140 may be configured for coupling in fluid communication with the fluid supply lumen 110. The fluid instillation reservoir 142 may be positioned in fluid communication with the positive-pressure source 140. The positive-pressure source 140 may be adapted to be coupled in fluid communication with the fluid supply lumen 110 through the fluid instillation reservoir 142. Thus, the fluid instillation reservoir 142 may have an inlet for receiving positive pressure from the positive-pressure source 140 and an outlet for delivering the positive pressure and instillation fluid to the fluid supply lumen 110. Instillation fluid may be urged from the fluid instillation reservoir 142 by the positive pressure into the fluid supply lumen 110. The positive-pressure source 140, the fluid supply lumen 110, and the fluid instillation reservoir 142 may be fluidly coupled to one another in any suitable manner, such as, without limitation, through tubing or piping that may be coupled with adhesives, bonding, welding, couplers, unions, or interference fit.

As shown in FIG. 1, a portable pump 156 may provide both the reduced-pressure source 136 and the positive-pressure source 140. For example, the pump 156 may include a suction port or pump inlet 158 and an exhaust port or pump outlet 160. The pump inlet 158 may provide the reduced-pressure source 136, and the pump outlet 160 may provide the positive-pressure source 140. The reduced-pressure valve 150 may be positioned in fluid communication between the reduced-pressure lumen 112 and the pump inlet 158. Reduced pressure from the reduced-pressure source 136 may be communicated to the reduced-pressure lumen 112 through the pump inlet 158 and the reduced-pressure valve 150. The positive-pressure valve 148 may be positioned in fluid communication between the fluid supply lumen 110 and the pump outlet 160. Positive pressure from the positive-pressure source 140 may be communicated to the fluid supply lumen 110 through the pump outlet 160 and the positive-pressure valve 148. Positive pressure applied to the dressing 114 may assist with communicating and distributing instillation fluid from the fluid instillation reservoir 142 to the dressing 114 and the tissue site 116. Fluid head, gravitational forces, and other factors may assist with communicating and distributing instillation fluid to the dressing 114 and the tissue site 116 with or without the application of positive pressure. Thus, some embodiments may not require the positive-pressure source 140.

In other embodiments, a first pump may provide the reduced-pressure source 136 and a second pump (not shown) may provide the positive-pressure source 140. Further, in other embodiments, the reduced-pressure source 136 may be any suitable device for providing reduced pressure, such as, for example, a wall suction source, a hand pump, or other source. Similarly, in other embodiments, the positive-pressure source 140 may be any suitable device for providing positive pressure, such as, for example, a compressor, compressed air cylinder, peristaltic pump, or similar source.

The reduced-pressure source 136 and the positive-pressure source 140 may be configured to supply reduced pressure and positive pressure, respectively, to the dressing 114 and the tissue site 116 in any combination or manner suitable for a particular application. For example, the controller 146 may be electrically coupled in any suitable manner to the reduced-pressure valve 150, the positive-pressure valve 148, and the pump 156. The controller 146 may include software or user programmable settings for controlling the reduced-pressure valve 150, the positive-pressure valve 148, and the pump 156 in relation to one another. In embodiments using a first pump for the reduced-pressure source 136 and a second pump for the positive-pressure source 140, the first pump and the second pump may be controlled by the controller 146 analogous to the reduced-pressure valve 150 and the positive-pressure valve 148 as described herein. In other embodiments, the reduced-pressure valve 150 and the positive-pressure valve 148 may be opened and closed or otherwise controlled manually by a user. Similarly, the pump 156 may be manually controlled.

In some embodiments, the pump 156 may be activated and the reduced-pressure valve 150 may be opened or otherwise activated simultaneously with the positive-pressure valve 148 for communicating reduced pressure and positive pressure to the tissue site 116 at the same time. The activation of the reduced-pressure valve 150 for reduced pressure delivery and the positive-pressure valve 148 for positive pressure delivery may be intermittent or continuous. In other embodiments, the pump 156 may activated and the reduced-pressure valve 150 may be configured to operate cyclically in relation to the positive-pressure valve 148. For example, the reduced-pressure valve 150 may be opened or activated to communicate reduced pressure to the dressing 114 and the tissue site 116 during a reduced-pressure therapy cycle. During the reduced-pressure therapy cycle, the positive-pressure valve 148 may be closed or inactive. During an instillation fluid cycle, the positive-pressure valve 148 may be opened or activated to communicate positive pressure to the dressing 114 and the tissue site 116. The reduced-pressure valve 150 may be closed or inactive during the instillation fluid cycle. The reduced-pressure valve 150 may have a reduced-pressure vent 162 for venting reduced pressure to the atmosphere, for example, when the reduced-pressure valve 150 is closed or inactive during the instillation fluid cycle. Similarly, the positive-pressure valve 148 may have a positive-pressure vent 164 for venting positive pressure to the atmosphere, for example, when the positive-pressure valve 148 is closed or inactive during the reduced-pressure therapy cycle.

Reduced pressure may be applied to the tissue site 116 from the reduced-pressure source 136 to promote removal of ascites, exudates, or other fluids from the tissue site 116. The fluid removed from the tissue site 116 by operation of reduced pressure being applied to the tissue site 116 may be about 5 liters or more per day. Further, reduced pressure may be applied to stimulate the growth of additional tissue, and to enhance the distribution of instillation fluids to the tissue site 116, if applicable. In the case of a wound at the tissue site 116, the growth of granulation tissue, removal of exudates, or removal of bacteria may promote healing. In the situation of a non-wounded or non-defective tissue, reduced pressure may promote the growth of tissue that may be harvested and transplanted to another tissue site.

As used herein, "reduced pressure" may refer to a pressure less than the ambient pressure at a tissue site being subjected to treatment. In some embodiments, the reduced pressure may be less than the atmospheric pressure. The reduced pressure may also be less than a hydrostatic pressure at a tissue site. Unless otherwise indicated, values of pressure stated herein are gauge pressures. The reduced pressure delivered may be a constant pressure, varied pressure, intermittent pressure, or continuous pressure. Although the terms "vacuum" and "negative pressure" may be used to describe the pressure applied to a tissue site, the actual pressure applied to the tissue site may be more than the pressure normally associated with a complete vacuum. An increase in reduced pressure may correspond to a reduction in pressure (more negative relative to ambient pressure) and a decrease in reduced pressure may correspond to an increase in pressure (less negative relative to ambient pressure). While the amount and nature of reduced pressure applied to a tissue site may vary according to the application, in some embodiments, the reduced pressure may be between about -5 mm Hg to about -500 mm Hg. In other embodiments, the reduced pressure may be between about -100 mm Hg to about -200 mm Hg. In yet other embodiments, the reduced pressure may be between about -50 mm Hg to about -300 mm Hg.

Further, in some embodiments, components of the system 102, such as, without limitation, the reduced-pressure source 136, the therapy device 104, or the controller 146, may include preset selectors for -100 mm Hg, -125 mm Hg, and -150 mm Hg of reduced pressure. Further, the system 102 may also include a number of alarms, such as, for example, a blockage alarm, a leakage alarm, or a battery-low alarm.

The dressing sealing member 106 may be adapted to cover at least a portion of the dressing 114 and the tissue site 116, and to provide a fluid seal and a sealed space 166 about the tissue site 116 or between the dressing sealing member 106 and the tissue site 116. A portion of the dressing sealing member 106 may overlap or cover tissue surrounding the tissue site 116, such as the epidermis 118. The dressing 114 may be sized or otherwise adapted to be positioned in the sealed space 166, and may be secured at an external opening 168 of the tissue site 116. The external opening 168 may provide access to the tissue site 116 from an exterior of the tissue site 116. The dressing sealing member 106 may provide a fluid seal, for example, at, over, or covering the external opening 168.

The dressing sealing member 106 may be formed from any material that may allow for a fluid seal, such as, for example, a liquid impermeable material. A fluid seal may be a seal adequate to maintain reduced pressure, if applicable, at a desired site. The dressing sealing member 106 may comprise, for example, one or more of the following materials: hydrophilic polyurethane; cellulosics; hydrophilic polyamides; polyvinyl alcohol; polyvinyl pyrrolidone; hydrophilic acrylics; hydrophilic silicone elastomers; an INSPIRE 2301 material from Expopack Advanced Coatings of Wrexham, United Kingdom having, for example, a moisture vapor transmission rate or MVTR (inverted cup technique) of 14400 g/m2/24 hours and a thickness of about 30 microns; a thin, uncoated polymer drape; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; polyurethane (PU); EVA film; copolyester; silicones; a silicone drape; a 3M Tegaderm® drape; a polyurethane (PU) drape such as one available from Avery Dennison Corporation of Pasadena, California; polyether block polyamide copolymer (PEBAX), for example, from Arkema, France; EXPOPACK 2327; or other appropriate material.

The dressing sealing member 106 may be vapor permeable and liquid impermeable, thereby allowing vapor and inhibiting liquids from exiting the sealed space 166. In some embodiments, the dressing sealing member 106 may be a flexible, breathable film, membrane, or sheet having a high MVTR of, for example, at least about 300g/m2 per 24 hours. The use of a high MVTR material for the dressing sealing member 106 may permit moisture vapor to pass through the dressing sealing member 106, external to the sealed space 166, while maintaining the fluid seal described above. In other embodiments, a low or no vapor transfer drape might be used. In some embodiments, the dressing sealing member 106 may comprise a range of medically suitable films having a thickness between about 15 microns (µm) to about 50 microns (µm).

In some embodiments, an attachment device or interface adhesive 172 may be adapted to be positioned between the dressing sealing member 106 and the tissue site 116. For example, the interface adhesive 172 may be positioned on or applied to an interior facing side of the dressing sealing member 106 for facing the tissue site 116. In some embodiments, the dressing sealing member 106 may be sealed directly against tissue surrounding the tissue site 116, such as the epidermis 118, by the interface adhesive 172. In other embodiments, the interface adhesive 172 may seal the dressing sealing member 106 against a gasket or drape (not shown) adapted to be positioned between the interface adhesive 172 and the epidermis 118.

The interface adhesive 172 may be a medically-acceptable adhesive and may take numerous forms, such as an adhesive sealing tape, drape tape, paste, hydrocolloid, hydrogel, or other suitable sealing device. The interface adhesive 172 may also be flowable. Further, the interface adhesive 172 may comprise, without limitation, an acrylic adhesive, rubber adhesive, high-tack silicone adhesive, polyurethane, or other adhesive substance. In some embodiments, the interface adhesive 172 may be a pressure-sensitive adhesive comprising an acrylic adhesive with coat weight, for example, of about 15 grams/m2 (gsm) to about 70 grams/m2 (gsm). In some embodiments, the interface adhesive 172 may be continuous or discontinuous.

Referring to FIGS. 2-3, in some illustrative embodiments, the dressing 114 may include a manifold 180 and a paddle 184. The paddle 184 may also be referred to as a blade 184. In some embodiments, the manifold 180 may be an elongate manifold 180 as shown in FIGS. 2-3. For example, the manifold 180 may have a width normal or perpendicular to the length 196. The length 196 may be greater than the width of the manifold 180. In some embodiments, the length 196 may be about four times greater, or more, than the width of the manifold 180. Further, in some embodiments, the width of the manifold 180 may be less than 8 millimeters. In other embodiments, the manifold 180 may have any suitable shape. Further, in some embodiments, the dressing 114 may include a tail 186. Even further, in some embodiments, the dressing 114 may include a fluid instillation pathway 190, and the fluid supply lumen 110 may be adapted to be coupled in fluid communication between the fluid instillation pathway 190 and the fluid instillation reservoir 142.

The manifold 180 may be adapted to distribute fluid and may include an insertion end 192, a connection end 194, and a length 196 between the insertion end 192 and the connection end 194. The insertion end 192 of the manifold 180 may be adapted for insertion at the tissue site 116. The reduced-pressure lumen 112 may be in fluid communication with the manifold 180, and may be coupled at the connection end 194 of the manifold 180, or another location. The reduced-pressure source 136 may be for coupling in fluid communication with the reduced-pressure lumen 112. The reduced-pressure lumen 112 may have a length that is fluidly isolated from a length of the fluid supply lumen 110. In other embodiments, components of the dressing 114 may be omitted or added as described herein.

The manifold 180 may be formed from any manifold material or flexible bolster material that provides a vacuum space, or treatment space, such as, for example, a porous and permeable foam or foam-like material, a member formed with pathways, a graft, or a gauze. In some embodiments, any material or combination of materials may be used as a manifold material for the manifold 180 provided that the manifold material is operable to distribute or collect fluid across a tissue site. For example, the term manifold may refer to a substance or structure capable of delivering fluids to or removing fluids from across a tissue site through a plurality of pores, pathways, or flow channels. The plurality of pores, pathways, or flow channels may be interconnected to improve distribution of fluids provided to and removed from an area around the manifold. Examples of such manifolds may include, without limitation, devices that have structural elements arranged to form flow channels, cellular foam, such as open-cell foam, porous tissue collections, and liquids, gels, and foams that include or cure to include flow channels. Further, the manifold 180 may be biocompatible. In some embodiments, the manifold 180 may comprise a porous, hydrophobic material. In such an embodiment, the hydrophobic characteristics of the manifold 180 may prevent the manifold 180 from directly absorbing fluid, but may allow the fluid to pass through.

In some embodiments, the manifold 180 may be a reticulated, open-cell polyurethane or polyether foam that is fluid permeable. One such material may be the VAC® GranuFoam® material available from Kinetic Concepts, Inc. of San Antonio, Texas. However, a material with a higher or lower density than GranuFoam® material may be desirable for the manifold 180 depending on the application. Among the many possible materials, the following may be used without limitation: GranuFoam® material; Foamex® technical foam (www.foamex.com); LIBELTEX DRY WEB; LIBELTEX TDL2; LIBELTEX TL4; a molded bed of nails structure; a patterned grid material, such as those manufactured by Sercol Industrial Fabrics; 3D textiles, such as those manufactured by Baltex of Derby, U.K.; a mass of filaments that may be adapted to provide a laminar fluid flow; a unidirectional manifold structure, such as a bundle of longitudinal filaments; a bundle of longitudinal filaments oriented substantially collinear to a desired direction of fluid flow; an array of polyamide monofilaments; a gauze; a flexible channel-containing member; and a graft.

In other embodiments, the manifold 180 may comprise a material including closed cells. The closed cells may not be fluidly connected to adjacent cells in the manifold 180. The closed cells may be selectively disposed in the manifold 180 to, for example, prevent transmission of fluids through perimeter surfaces of the manifold 180. Other layers may be included in or on the manifold 180, such as absorptive materials, wicking materials, hydrophobic materials, and hydrophilic materials. In some embodiments, the manifold 180 may be enhanced with ionic silver and anti-microbial agents.

In some embodiments, the manifold 180 may be covered, surrounded, or encapsulated by a liquid impermeable material. For example, in some embodiments, the manifold 180 may be covered, surrounded, or encapsulated by a manifold film 198, and the manifold film 198 may be formed of a liquid impermeable material. The liquid impermeable material may be substantially liquid impermeable or entirely liquid impermeable. Further, in some embodiments, the manifold film 198 may include a plurality of manifold film fenestrations 202 disposed through the manifold film 198. The manifold film fenestrations 202 may be in fluid communication with the manifold 180.

In some embodiments, the manifold film 198 may comprise a non-adherent material, such as a medical drape, capable of inhibiting tissue from adhering to the medical drape. In some embodiments, the manifold film 198 may comprise a breathable polyurethane film. Further, in some embodiments, manifold film 198 may comprise any of the materials recited above for the dressing sealing member 106. Even further, in some embodiments, at least a portion of the manifold film 198 may be hydrophilic. For example, the manifold film 198 may comprise a plasma treatment that may impart hydrophilic properties to the manifold film 198. Such hydrophilic properties may encourage or improve fluid coverage over a greater surface of the manifold film 198 rather than, for example, being drawn to a low point at the tissue site 116 by operation of gravitational forces.

In some embodiments, the manifold 202 may be positioned between a first film layer 206 and a second film layer 210. The first film layer 206 and the second film layer 210 may define the manifold film 198 that may cover, surround, or encapsulate the manifold 180. For example, a periphery 212 of the second film layer 210 may be coupled to the first film layer 206 around the manifold 180 or at opposing sides of the manifold 180. The first film layer 206 may be coupled to the second film layer 210 in any suitable manner, such as, without limitation, welding, bonding, adhesives, cements, or similar bonding devices. The first film layer 206 and the second film layer 210 may each be comprised of a liquid impermeable material, or any of the materials recited above for the manifold film 198.

The first film layer 206 may include a plurality of first film layer fenestrations 214 that may be disposed through the first film layer 206 and may be positioned in fluid communication with the manifold 180. Similarly, the second film layer 210 may include a plurality of second film layer fenestrations 216 that may be disposed through the second film layer 210 and maybe positioned in fluid communication with the manifold 180.

Continuing with FIGS. 2-3, the paddle 184 may be positioned in fluid communication with the manifold 180. The paddle 184 may extend outward from the manifold 180 and normal relative to the length 196 of the manifold 180. Further, the paddle 184 may be positioned proximate to the insertion end 192 of the manifold 180. In embodiments of the tissue site 116 including the abdominal cavity 124, the manifold 180 may be positioned adjacent to, against, or supported by the abdominal contents 126, while the paddle 184 may be positioned at the first paracolic gutter 128 or the second paracolic gutter 130. Other embodiments are possible. For example, the insertion end 192 of the manifold 180 and the paddle 184 may be positioned at or in the first paracolic gutter 128 or the second paracolic gutter 130. Further, in some embodiments, more than one dressing 114 may be positioned at the tissue site 116 as desired.

In some embodiments, the dressing 114 may include two of the paddles 184 as shown in FIG. 2. However, in other embodiments, the dressing 114 may include one of the paddles 184, more than two of the paddles 184, or any number of the paddles 184 as desired. For example, as shown in FIG. 2, the paddle 184 may be a first paddle or a paddle 184a, and the dressing 114 may further include another of the paddles 184, such as a second paddle or a paddle 184b. The paddle 184a and the paddle 184b may extend outward from the sides of the manifold 180 along the length 196 of the manifold 180. Further, the paddle 184a and the paddle 184b may extend outward from opposing sides of the manifold 180 such that the manifold 180 may be substantially centered between the paddle 184a and the paddle 184b. Other configurations for positioning one or more of the paddle 184 relative to the manifold 180 are possible.

In some illustrative embodiments, the paddle 184, also being referred to as the blade 184, may include a paddle film layer 220 or film layer 220, a paddle surface 224 or surface 224, and at least one surface protrusion 228. The surface protrusion 228 may also be referred to as a surface feature 228. Any feature described herein in connection with the paddle 184 may be applicable to the blade 184, and may have an analogous structure and operation. Similarly, any feature described herein in connection with the blade 184 may be applicable to the paddle 184, and may have an analogous structure and operation.

The manifold 180 may be adapted to distribute reduced pressure to the paddle 184 and the at least one surface protrusion 228. In some embodiments, the paddle 184 may include a plurality of paddle fenestrations 230 disposed through the paddle 184. In other embodiments, components of the paddle 184 may be omitted or added as described herein.

As described above, in some embodiments, the manifold 180 may be positioned between the first film layer 206 and the second film layer 210 with the periphery 212 of the second film layer 210 being coupled to the first film layer 206 around or at opposing sides of the manifold 180. In such embodiments, the paddle 184 may include a portion of the first film layer 206 extending beyond the periphery 212 of the second film layer 210.

The paddle 184 may be configured to collapse. For example, the paddle 184 may be configured to collapse about the manifold 180 when the dressing 114 is extracted from the tissue site 116 through the external opening 168 at the tissue site 116. For example, the paddle 184 may include a paddle width 234 and a paddle taper 236. The paddle taper 236 may provide a slope positioned between the paddle width 234 and the tail 186 of the dressing 114. The paddle taper 236 may define a gradual reduction in size or width of the paddle 184 or the dressing 114 between the paddle width 234 and the tail 186 of the dressing 114. In such a configuration, the paddle taper 236 may engage the external opening 168 upon extraction from the tissue site 116, thereby collapsing or folding the paddle 184 to a shape and size capable of being drawn through the external opening 168. Other configurations are possible as described herein for enhancing the ability of the paddle 184 to collapse or otherwise deform sufficiently in size and shape for removal from the tissue site 116 through the external opening 168.

The paddle film layer 220 may extend outward from the manifold 180, and in some embodiments, may extend normal relative to the length 196 of the manifold 180. A portion of the paddle film layer 220 may cross or cover the manifold 180. The paddle surface 224 may be defined by the paddle film layer 220, and the paddle surface 224 may be in fluid communication with the manifold 180. Further, the paddle surface 224 and the at least one surface protrusion 228 of the paddle 184 may each be in fluid communication with the manifold 180.

Components of the paddle 184, such as the paddle film layer 220, may be comprised of, for example, a liquid impermeable material, a polyurethane film, or any of the materials recited above for the manifold film 198. Further, in some embodiments, the paddle film layer 220 may be a single layer, such as a single layer of a liquid impermeable film. The use of a single layer for the paddle film layer 220 may enhance the ability of the paddle 184 to collapse or otherwise reduce in size and shape for removal from the tissue site 116 as described above.

The at least one surface protrusion 228 may be in fluid communication with the manifold 180, and may include a protrusion length 240 and a protrusion height 242. The protrusion length 240 may extend outward from the manifold 180 in a lateral direction relative to the length 196 of the manifold 180. A portion of the protrusion length 240 may intersect or cross a portion of the manifold 180. The protrusion height 242 may extend normal to the protrusion length 240. In some embodiments, the protrusion height 242 may extend outward from the paddle surface 224 of the paddle 184 and normal to the paddle surface 224. Further, the protrusion length 240 may extend along the paddle surface 224, which may be parallel to the paddle surface 224. The protrusion length 240 may be elongate and substantially continuous. In some embodiments, the at least one surface protrusion 228 may be carried by the paddle film layer 220.

In some embodiments, the at least one surface protrusion 228 may include a fold 246 as shown in FIG. 3. The fold 246 may be positioned on the paddle film layer 220, and may be formed from a substrate material of the paddle film layer 220. For example, a portion of the paddle film layer 220 may be pinched and coupled together to form the fold 246. In some embodiments, the at least one surface protrusion 228 may be, without limitation, a pinched, welded, extruded, embossed, or thermoformed portion of a substrate material of the paddle 184 or the paddle film layer 220 that may be shaped or otherwise adapted to form a fluid communication pathway 248 across, with, or relative to the manifold 180 and the tissue site 116. The fluid communication pathway 248 may also be referred to as a fluid channel 248. The surface protrusion 228 or surface feature 228 may also be a recess or depression, in some embodiments. Further, in some embodiments, the at least one surface protrusion 228 may be a separately formed component or layer that is coupled, welded, or otherwise adhered to the paddle 184. In some embodiments, the at least one surface protrusion 228 may be a plurality of surface protrusions 228, which may be gathered, coupled, or otherwise directed toward the manifold 180. Thus, the at least one surface protrusion 228 may be configured to direct the flow of fluid to and from the tissue site 116, while minimizing any waste of fluid, such as the instillation fluid described below. Further, the at least one surface protrusion 228 may provide an intentional path of least resistance, such as the fluid channel 248, for the fluid to flow. The fluid channel 248 and other fluid channels 248 may be formed by deformation or collapse of the at least one surface protrusion 228 when under reduced pressure. However, as described further below, the fluid channels 248 may be configured to remain open under reduced pressure.

Continuing with FIGS. 2-3, the tail 186 may be positioned proximate to the connection end 194 of the manifold 180. Further, the tail 186 may be adapted to be positioned proximate the external opening 168 at the tissue site 116 as shown in FIG. 1. In some embodiments, the tail 186 may be coupled to the connection end 194 of the manifold 180, and configured to be accessible through the external opening 168 at the tissue site 116. In some embodiments, the tail 186 may be adapted to extend through the external opening 168 at the tissue site 116, and the sealing member 106 may positioned, coupled, or sealed about the tail 186 and the external opening 168. The tail 186 may be formed as part of the manifold film 198, the first film layer 206, or the second film layer 210 that may, for example, be adapted in any suitable manner to position or couple the fluid supply lumen 110 and the reduced-pressure lumen 112 in fluid communication with the manifold 180. For example, the tail 186 may be a portion of the manifold film 198, the first film layer 206, or the second film layer 210 that may be positioned at or extending beyond the connection end 194 of the manifold 180 for fluidly connecting to the fluid supply lumen 110 and the reduced-pressure lumen 112.

In some embodiments, the fluid supply lumen 110 and the reduced-pressure lumen 112 may be coupled at the tail 186, or form a part of the tail 186. Similar to the tail 186, the fluid supply lumen 110 and the reduced-pressure lumen 112 may be configured to be accessible at the external opening 168 of the tissue site 116. In some embodiments, the fluid supply lumen 110 and the reduced-pressure lumen 112 may pass through the tail 186. Further, in some embodiments, the fluid supply lumen 110 and the reduced-pressure lumen 112 may be coupled to the tail 186 in a staggered or offset configuration to reduce the amount of material positioned at the tissue site 116.

**In** some embodiments, the fluid supply lumen 110 and the reduced-pressure lumen 112 may be color-coded. For example, the fluid supply lumen 110 may have a color distinguishable from a color associated with the reduced-pressure lumen 112. Such a color-coded configuration for the fluid supply lumen 110 and the reduced-pressure lumen 112 may assist the end user with properly positioning and connecting the dressing 114 for use.

The fluid instillation pathway 190 may be positioned along the manifold 180, for example, along the length 196 of the manifold 180, and in fluid communication with the paddle 184. For example, the fluid instillation pathway 190 may be in fluid communication with the paddle surface 224 of the paddle 184 and the at least one surface protrusion 228. A barrier 250 may be positioned between the manifold 180 and the fluid instillation pathway 190. The fluid instillation pathway 190 may be defined between the barrier 250 and an instillation film layer 254. In some embodiments, the instillation film layer 254 may include a plurality of instillation film layer fenestrations 262 disposed through the instillation film layer 254 in fluid communication with the fluid instillation pathway 190 and the paddle 184.

In some embodiments, a plurality of barrier fenestrations 258 may be disposed through the barrier 254 in fluid communication with the manifold 180 and the fluid instillation pathway 190. However, the barrier 250 may separate the manifold 180 from direct physical contact with the fluid instillation pathway 190. Such separation of direct physical contact of the manifold 180 from the fluid instillation pathway 190 may provide a preferential path of least resistance through the fluid instillation pathway 190 for instillation fluid to follow during delivery to the tissue site 116. Thus, the configuration of the fluid instillation pathway 190 and the barrier 250 may prevent any fluid, such as waste fluid, in the manifold 180 from being forced back into the tissue site 116 during delivery of instillation fluid to the tissue site 116.

In some embodiments, a portion of the second film layer 210 or the manifold film 198 may define the barrier 250. In such embodiments, the barrier fenestrations 258 may be analogous to the manifold film fenestrations 202 or the second film layer fenestrations 216. For example, in embodiments where the manifold 180 may be positioned between the first film layer 206 and the second film layer 210, the fluid instillation pathway 190 may be defined between the second film layer 210 and the instillation film layer 254. In such embodiments, a periphery 264 of the instillation film layer 254 may be coupled, for example, by a weld 268, at or proximate to the periphery 212 of the second film layer 210 to define the fluid instillation pathway 190. The weld 268 may join or couple both the periphery 212 of the second film layer 210 and the periphery 264 of the instillation film layer 254 to the first film layer 206. Any suitable coupling device, such as adhesives, may be used for joining components of the dressing 114 as described above. Further, in such embodiments, the paddle 184 may extend beyond the periphery 212 of the second film layer 210 and the periphery 264 of the instillation film layer 254.

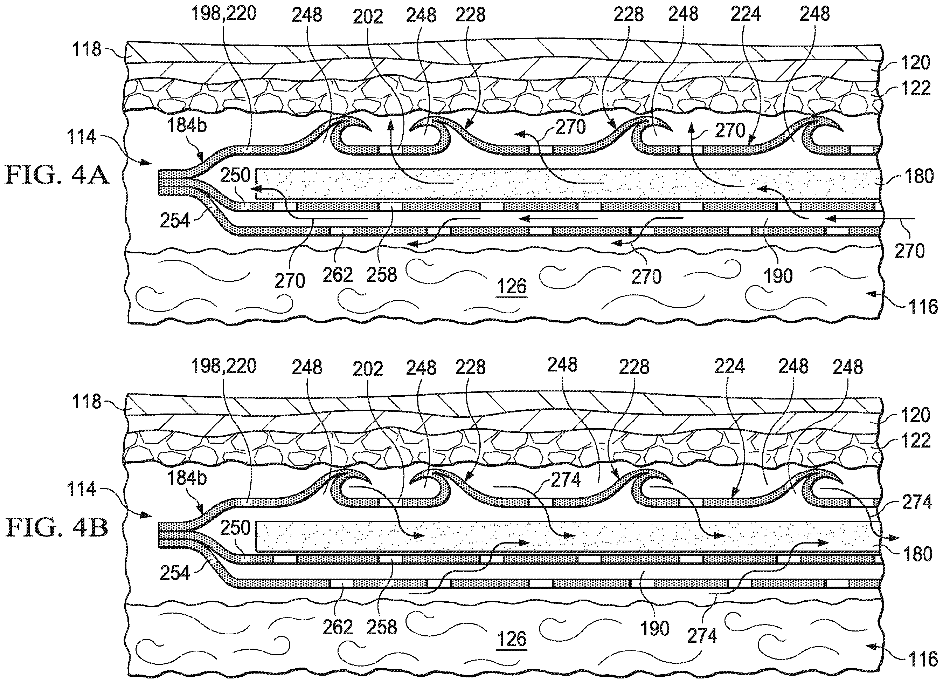

Referring to FIGS. 4A-4B, the fluid instillation pathway 190 may permit various fluids, such as, without limitation, medicines, irrigation fluids, instillation fluids, or therapeutic fluids, to be delivered to the tissue site 116 as shown by delivery arrows 270 in FIG. 4A. After being delivered to the tissue site 116, these fluids or other fluids may be removed or extracted from the tissue site 116 as shown by extraction arrows 274. Instillation fluid delivered to the fluid instillation pathway 190 may contact the manifold 180 during delivery to the tissue site 116 or extraction from the tissue site 116. Such instillation fluid may contact the manifold 180 and may dilute any exudate or other waste fluid from the tissue site 116 that may be present in and around the manifold 180. Dilution of the exudate or other waste fluid from the tissue site 116 may reduce the viscosity of this waste fluid, thereby enhancing the ability of the waste fluid to flow and to be extracted from the tissue site 116. Further, the ability to dilute the waste fluid with instillation fluid or other therapeutic fluid may reduce the amount or concentration of waste fluid that may be forced back into the tissue site 116 upon removal of the dressing 114 from the tissue site 116. The barrier fenestrations 250 may enhance the dilution of the waste fluid from the tissue site 116.

Referring generally to FIGS. 1-4B, in some illustrative embodiments of operation of the system 102, the dressing 114 may be disposed at or within the tissue site 116, such as the abdominal cavity 124. If sizing the dressing 114 is necessary, excess portions of the dressing 114 may be removed, for example, by cutting or tearing through the dressing 114.

The dressing 114 may be positioned adjacent to or in contact with the tissue site 116, and in some embodiments, the abdominal cavity 124. In some embodiments, the paddle 184 of the dressing 114 may be positioned in or proximate to the first paracolic gutter 128 and the second paracolic gutter 130. In other embodiments, the paddle 184 and the insertion end 192 of the manifold 180 may be positioned in or proximate to the first paracolic gutter 128 or the second paracolic gutter 130. Additional dressings 114 may be used and positioned as desired, for example, on opposite sides of the abdominal contents 126. When deployed, the dressing 114 may cover all exposed viscera and may separate the viscera from contact with the walls of the abdominal cavity 124. The dressing 114 may be sized and shaped to permit such coverage. The dressing sealing member 106 may be positioned and fluidly sealed about the tissue site 116 with the interface adhesive 172 as described above. The tail 186, the fluid supply lumen 110, and the reduced-pressure lumen 112 may be accessible through the external opening 168 of the tissue site 116 for connection to the therapy device 104 or other components.

Activating the reduced-pressure source 136 may provide reduced pressure to the tissue site 116 through the reduced-pressure lumen 112, the manifold 180, and the paddle 184. The instillation fluid reservoir 142 may provide instillation fluid to the fluid instillation pathway 190 through the fluid supply lumen 110, for example, by activating the positive-pressure source 140, or by operation of gravitational forces acting on the instillation fluid. Reduced pressure and instillation fluid may be provided to the dressing 114 simultaneously, at the same time, or cyclically, at alternate times. Further, reduced pressure and instillation fluid may be applied to the dressing 114 intermittently or continuously.

When the reduced-pressure source 136 is activated, the manifold 180 may distribute the reduced pressure to the paddle 184 and to the tissue site 116. As shown in FIG. 4B by the extraction arrows 274, fluid from the tissue site 116 may be drawn or extracted through or along the paddle surface 224 and the surface protrusions 228 toward the manifold 180. Fluid may be communicated from the paddle 184 to the manifold 180 where the fluid may be drawn into the reduced-pressure lumen 112 and the canister 138.

When under reduced pressure, the surface protrusions 228 may collapse, crease, or otherwise deform to define the fluid channels 248 that may be positioned to direct or provide fluid flow in the direction of the manifold 180. The fluid channels 248 may also be defined from positioning or compression of the dressing 114 at the tissue site 116, and thus, may be present without the application of reduced pressure and while instillation fluid is being provided to the tissue site 116. Deformation of the surface protrusions 228 is not required to form the fluid channels 248.

When the positive-pressure source 140 is activated or instillation fluid is otherwise being delivered to the dressing 114, the instillation fluid may pass into the fluid instillation pathway 190 as shown by the delivery arrows 270 in FIG. 4A. The instillation fluid may be communicated to the tissue site 116 through a combination of features, such as, for example, the instillation film layer fenestrations 262, the barrier fenestrations 258, the manifold film fenestrations 202, and the fluid channels 248. Once delivered to the tissue site 116, the instillation fluid may become comingled with, for example, previously instilled fluid, wound fluid, tissue fluid, and other fluid that may be considered waste fluid. When reduced pressure is being applied to the dressing 114, fluid from the tissue site 116 and any instillation fluid previously delivered to the tissue site 116 may be extracted through the manifold 180.

The dressing 114 may be removed from the tissue site 116 post-operatively in a non-invasive or less invasive manner with minimal surgical implications. For example, the external opening 168 at the tissue site 116 may have diameter between about 6 millimeters to about 10 millimeters, and in some embodiments, about 8 millimeters. The tail 186 may be accessed through or at the external opening 168, and the dressing 114 may be removed or pulled from the tissue site 116 by the tail 186 or other components of the dressing 114, such as the fluid supply lumen 110 or the reduced-pressure lumen 112. As the dressing 114 is drawn through the external opening 168, the paddle 184 may collapse, fold, or otherwise deform to a sufficient size and shape to permit withdrawal from the tissue site 116. Thus, the configuration of the dressing 114 may provide for drainage of the tissue site 116 without the need for further surgery when the dressing 114 requires removal. Further, in some embodiments, the elongate configuration of the manifold 180 and central positioning of the manifold 180 can minimize the amount of fluid retained by the manifold 180 and the dressing 114 in general, thereby reducing any tendency for waste fluid to be forced back into the tissue site 116 during removal of the dressing 114.

Continuing generally with FIGS. 1-4B, further described are methods for treating the tissue site 116. In some illustrative embodiments, a method for treating the tissue site 116 may include positioning the dressing 114 adjacent to the tissue site 116. The dressing 114 may include the manifold 180 and the paddle 184 that may extend outward from the manifold 180 and in fluid communication with the manifold 180. The paddle 184 may carry the at least one surface protrusion 228 on the paddle surface 224 of the paddle 184. Further, the method may include applying reduced pressure to the tissue site 116 through the manifold 180 and the paddle 184; moving a fluid from the tissue site 116 to the manifold 180 along the at least one surface protrusion 228 and the paddle surface 224 of the paddle 184; and extracting the fluid from the tissue site 116 through the manifold 180. In some embodiments, the tissue site 116 may be the abdominal cavity 124, and positioning the dressing 114 adjacent to the tissue site 116 may include placing the paddle 184 of the dressing 114 proximate to a paracolic gutter, such as the first paracolic gutter 128 or the second paracolic gutter 130, of the abdominal cavity 124.

In some embodiments, the manifold 180 may be an elongate manifold 180 that may include the insertion end 192 and the connection end 194. Further, the dressing 114 may include the tail 186 coupled at the connection end 194 of the elongate manifold 180, and the paddle 184 may be positioned proximate to the insertion end 192 of the manifold 180 and opposite the tail 186. In some embodiments, positioning the dressing 114 may include inserting the insertion end 192 at the tissue site 116, and positioning the tail 186 proximate to the external opening 168 at the tissue site 116.

In some embodiments, the method may include sealing the external opening 168 of the tissue site 116 at the tail 186 of the dressing 114. Further, in some embodiments, the method may include pulling the dressing 114 from the tissue site 116 by the tail 186. Further, in some embodiments, the method may include pulling the paddle 184 and the insertion end 192 of the manifold 180 through the external opening 168 at the tissue site 116. The paddle 184 may collapse upon being pulled through the external opening 168.

In some embodiments, the method may include positioning the fluid instillation pathway 190 in fluid communication with the paddle 184, and supplying instillation fluid to the tissue site 116 through the fluid instillation pathway 190 and the paddle 184. In some embodiments, the method may include supplying instillation fluid to the tissue site 116 through the paddle 184, and diluting the fluid from the tissue site 116 being extracted through the manifold 180.

In another illustrative embodiment, a method for treating the abdominal cavity 124 may include providing the manifold 180. The manifold 180 may include the insertion end 192, which may be adapted for insertion in the abdominal cavity 124. Further, the method may include providing the blade 184 positioned proximate to the insertion end 192 of the manifold 180. The blade 184 may include at least one surface feature 228 that may extend from the manifold 180 toward a periphery of the blade 184. The surface feature 228 may provide a fluid pathway 248 toward the manifold 180. Further, the method may include positioning the manifold 180 and the blade 184 in the abdominal cavity 124, coupling the reduced-pressure lumen 112 in fluid communication with the manifold 180, and coupling the reduced-pressure source 136 in fluid communication with the reduced-pressure lumen 112.

In some embodiments, the surface feature 228 may extend to the periphery of the blade 184. Further, in some embodiments, the blade 184 may not enclose foam, or may otherwise be free of foam. Further, in some embodiments, the blade 184 may be formed from a single layer of a substantially liquid impermeable film. Further, in some embodiments, the fluid pathway 248 may be configured to direct fluid along the surface 224 of the blade 184. Further, in some embodiments, the method may include extracting fluid from the abdominal cavity 124 through the fluid pathway 248 and the manifold 180.

## Claims

1. A dressing for treating a tissue site, comprising:
an elongate manifold (180) surrounded by a manifold film (198) and adapted to distribute fluid, the elongate manifold comprising an insertion end (192), a connection end (194), and a length between the insertion end and the connection end, the insertion end (192) adapted for insertion at the tissue site;
a paddle (184) positioned in fluid communication with the elongate manifold (180) proximate to the insertion end (192), the paddle comprising a paddle film layer (220) extending outward from the elongate manifold and normal relative to the length of the elongate manifold;
a tail (186) proximate to the connection end of the elongate manifold, the tail adapted to be positioned proximate to an external opening at the tissue site; and
a fluid instillation pathway (190) positioned along the length of the elongate manifold (180) and in fluid communication with the paddle (184).

2. The dressing of claim 1, wherein the manifold film (198) is formed from a liquid impermeable material.

3. The dressing of claim 1, wherein the manifold film (198) comprises a plurality of manifold film fenestrations (202) disposed through the manifold film, and wherein the manifold film fenestrations are in fluid communication with the elongate manifold (180).

4. The dressing of claim 1, wherein the paddle film layer (220) is formed from a liquid impermeable material.

5. The dressing of claim 4, wherein the paddle film layer (220) is a single layer of a liquid impermeable film.

6. The dressing of claim 1, wherein the paddle (184) comprises a plurality of paddle fenestrations disposed through the paddle.

7. The dressing of claim 1, wherein the paddle (184) comprises a paddle surface (224) and a plurality of surface protrusions (228), and wherein the paddle surface and the plurality surface protrusions are in fluid communication with the elongate manifold (180).

8. The dressing of claim 7, wherein the plurality of surface protrusions (228) comprise a protrusion height and a protrusion length, wherein the protrusion height extends outward from the paddle surface and normal to the paddle surface, and wherein the protrusion length extends along the paddle surface.

9. The dressing of claim 7, wherein the paddle surface is defined by the paddle film layer (220), and wherein the paddle film layer carries the plurality of surface protrusions (228).

10. The dressing of claim 1, further comprising a barrier (250) positioned between the elongate manifold (180) and the fluid instillation pathway (190).

11. The dressing of claim 10, wherein the fluid instillation pathway (190) is defined between the barrier (250) and an instillation film layer (254).

12. The dressing of claim 10, further comprising a plurality of barrier fenestrations disposed through the barrier (250) in fluid communication between the elongate manifold (180) and the fluid instillation pathway (190).

13. The dressing of claim 10, wherein at least a portion of the manifold film (198) defines the barrier.

14. The dressing of claim 1, wherein the tail (186) is coupled to the connection end (194) of the elongate manifold (180) and configured to be accessible through the external opening at the tissue site.

## Patentansprüche

1. Verband zur Behandlung einer Gewebestelle, umfassend:
einen langgestreckten Verteiler (180), der von einem Verteilerfilm (198) umgeben und zum Verteilen von Fluid angepasst ist, wobei der langgestreckte Verteiler ein Einschubende (192), ein Verbindungsende (194) und eine Länge zwischen dem Einschubende und dem Verbindungsende umfasst, wobei das Einschubende (192) zum Einführen an der Gewebestelle geeignet ist;
ein Paddel (184), das in Fluidverbindung mit dem langgestreckten Verteiler (180) nahe dem Einschubende (192) positioniert ist, wobei das Paddel eine Paddel-Filmschicht (220) umfasst, die sich vom langgestreckten Verteiler nach außen und senkrecht zur Länge des langgestreckten Verteilers erstreckt;
einen Schwanz (186) nahe dem Verbindungsende des langgestreckten Verteilers, wobei der Schwanz geeignet ist, nahe an einer äußeren Öffnung an der Gewebestelle positioniert zu werden; und
einen Fluid-Instillationspfad (190), der entlang der Länge des länglichen Verteilers (180) und in Fluidverbindung mit dem Paddel (184) positioniert ist.

2. Verband nach Anspruch 1, wobei der Verteilerfilm (198) aus einem flüssigkeitsundurchlässigen Material gebildet ist.

3. Verband nach Anspruch 1, wobei der Verteilerfilm (198) eine Vielzahl von Verteilerfilm-Fenstern (202) umfasst, die durch den Verteilerfilm angeordnet sind, und wobei die Verteilerfilm-Fenster in Fluidverbindung mit dem länglichen Verteiler (180) stehen.

4. Verband nach Anspruch 1, wobei die Paddelfilmschicht (220) aus einem flüssigkeitsundurchlässigen Material gebildet ist.

5. Verband nach Anspruch 4, wobei die Paddelfilmschicht (220) eine einzelne Schicht eines flüssigkeitsundurchlässigen Films ist.

6. Verband nach Anspruch 1, wobei das Paddel (184) eine Vielzahl von Paddelfenstern umfasst, die durch das Paddel angeordnet sind.

7. Verband nach Anspruch 1, wobei das Paddel (184) eine Paddeloberfläche (224) und eine Vielzahl von Oberflächenvorsprüngen (228) umfasst, und wobei die Paddeloberfläche und die Vielzahl von Oberflächenvorsprüngen in Fluidverbindung mit dem länglichen Verteiler (180) stehen.

8. Verband nach Anspruch 7, wobei die Vielzahl von Oberflächenvorsprüngen (228) eine Vorsprungshöhe und eine Vorsprungslänge umfasst, wobei sich die Vorsprungshöhe von der Paddeloberfläche nach außen und senkrecht zur Paddeloberfläche erstreckt und wobei sich die Vorsprungslänge entlang der Paddeloberfläche erstreckt.

9. Verband nach Anspruch 7, wobei die Paddeloberfläche durch die Paddelfilmschicht (220) definiert ist, und wobei die Paddelfilmschicht die Vielzahl von Oberflächenvorsprüngen (228) trägt.

10. Verband nach Anspruch 1, weiter umfassend eine Barriere (250), die zwischen dem länglichen Verteiler (180) und dem Fluid-Instillationsweg (190) angeordnet ist.

11. Verband nach Anspruch 10, wobei der Fluid-Instillationsweg (190) zwischen der Barriere (250) und einer Instillationsfilmschicht (254) definiert ist.

12. Verband nach Anspruch 10, weiter umfassend eine Vielzahl von Barrierefenstern, die durch die Barriere (250) in Fluidverbindung zwischen dem länglichen Verteiler (180) und dem Fluid-Instillationsweg (190) angeordnet sind.

13. Verband nach Anspruch 10, wobei mindestens ein Teil der Verteilerfolie (198) die Barriere definiert.

14. Verband nach Anspruch 1, wobei der Schwanz (186) mit dem Verbindungsende (194) des länglichen Verteilers (180) gekoppelt und so konfiguriert ist, dass er durch die äußere Öffnung an der Gewebestelle zugänglich ist.

## Revendications

1. Pansement pour traiter un site tissulaire, comprenant :
un collecteur allongé (180) entouré par un film de collecteur (198) et adapté à distribuer un fluide, le collecteur allongé comprenant une extrémité d'insertion (192), une extrémité de raccordement (194) et une longueur entre l'extrémité d'insertion et l'extrémité de raccordement, l'extrémité d'insertion (192) étant adaptée à être insérée au niveau du site tissulaire ;
une languette (184) placée en communication fluidique avec le collecteur allongé (180) à proximité de l'extrémité d'insertion (192), la languette comprenant une couche de film de languette (220) s'étendant vers l'extérieur à partir du collecteur allongé et perpendiculaire par rapport à la longueur du collecteur allongé ;
une queue (186) à proximité de l'extrémité de raccordement du collecteur allongé, la queue étant adaptée à être positionnée à proximité d'une ouverture externe au niveau du site tissulaire ; et
une voie de passage d'instillation de fluide (190) positionnée sur la longueur du collecteur allongé (180) et en communication fluidique avec la languette (184).

2. Pansement selon la revendication 1, dans lequel le film de collecteur (198) est formé en un matériau imperméable aux liquides.

3. Pansement selon la revendication 1, dans lequel le film de collecteur (198) comprend une pluralité de fenêtres de film de collecteur (202) disposées à travers le film de collecteur et dans lequel les fenêtre de film de collecteur sont en communication fluidique avec le collecteur allongé (180).

4. Pansement selon la revendication 1, dans lequel la couche de film de languette (220) est formée en un matériau imperméable aux liquides.

5. Pansement selon la revendication 4, dans lequel la couche de film de languette (220) est une couche unique d'un film imperméable aux liquides.

6. Pansement selon la revendication 1, dans lequel la languette (184) comprend une pluralité de fenêtres de languette disposées à travers la languette.

7. Pansement selon la revendication 1, dans lequel la languette (184) comprend une surface de languette (224) et une pluralité de saillies de surface (228), et dans lequel la surface de languette et les saillies de surface sont en communication fluidique avec le collecteur allongé (180).

8. Pansement selon la revendication 7, dans lequel la pluralité de saillies de surface (228) comprend une hauteur de saillie et une longueur de saillie, la hauteur de saillie s'étendant vers l'extérieur de la surface de la languette et perpendiculaire à la surface de la languette.

9. Pansement selon la revendication 7, dans lequel la surface de la languette est définie par la couche de film de languette (220), et dans lequel la couche de film de languette porte la pluralité de saillies de surface (228).

10. Pansement selon la revendication 1, comprenant en outre une barrière (250) positionnée entre le collecteur allongé (180) et la voie de passage d'instillation de fluide (190).

11. Pansement selon la revendication 10, dans lequel la voie de passage d'instillation de fluide (190) est définie entre la barrière (250) et une couche de film d'instillation (254).

12. Pansement selon la revendication 10, comprenant en outre une pluralité de fenêtres de barrière disposées à travers la barrière (250) en communication fluidique entre le collecteur allongé (180) et la voie de passage d'instillation de fluide (190).

13. Pansement selon la revendication 10, dans lequel au moins une partie du film de collecteur (198) définit la barrière.

14. Pansement selon la revendication 1, dans lequel la queue (186) est couplée à l'extrémité de raccordement (194) du collecteur allongé (180) et configurée pour être accessible via l'ouverture externe au niveau du site tissulaire.
